# EUROPEAN PATENT APPLICATION

(11) **EP 2 589 666 A1**
(43) Date of publication of application: **08.05.2013**
(21) Application number: 11800934.9
(22) Date of filing: 30.06.2011
(51) Int. Cl.: C12Q 1/68, C12N 15/00, G01N 33/50, G01N 33/574, G01N 33/68

(54) **ACF DETECTION METHOD**

(30) Priority: 30.06.2010 JP 2010148649
(71) Applicant: OLYMPUS CORPORATION, Shibuya-ku Tokyo 151-0072 (JP)
(72) Inventor: HORINO, Yoko, Tokyo 151-0072 (JP)
(74) Representative: Schicker, Silvia
(86) International application number: PCT/JP2011/064997
(87) International publication number: WO 2012/002472

(57) **Abstract**

The present invention provides a method for detecting ACF by analyzing a test region of large intestine tissue at the molecular level. Namely, the present invention relates to a method for detecting aberrant crypt foci (ACF) that comprises detecting one or more types of molecules for which ACF-specific expression increases selected from the group consisting of GSTp, iNOS, CD44, EGFR, COX2 and Fzd1 present in a test region of large intestine tissue; the aforementioned ACF detection method wherein the diameter of the test region is 0.5 mm or less; a an ACF detection marker that is GSTp, iNOS, CD44, EGFR, COX2 or Fzd1; and, a method for evaluating risk of colorectal cancer and colorectal adenoma in subjects based on the results of detecting ACF in a test region of large intestine tissue of the subjects using the aforementioned ACF detection method.

## Description

### TECHNICAL FIELD

The present invention relates to a method for detecting aberrant crypt foci (ACF) by using molecules specifically highly expressed for ACF.
The present application claims priority on the basis of Japanese Patent Application No. 2010-148649, filed in Japan on June 30, 2010, the contents of which are incorporated herein by reference.

### BACKGROUND ART

Colorectal cancer is the leading cause of death in Japan and the second leading cause of death in the U. S. Approximately 150,000 new cases of colorectal cancer are discovered in the U.S. each year, and more than 50, 000 of those cases die annually (as estimated by the American Cancer Society). On the other hand, since colorectal cancer frequently takes several tens of years to progress from a benign tumor to a malignant tumor, early risk assessment and discovery are expected to contribute to favorable prognosis and prevention.

Typical examples of colorectal adenoma and tumor screening methods currently employed at present include occult blood testing, barium enema, total colonoscopy and sigmoid colonoscopy. However, in the case of occult blood testing, for example, since there are cases in which blood is detected that is caused by factors other than an adenoma or tumor, it cannot be said to have high specificity for colorectal adenomas and tumors, and is susceptible to the occurrence of false positives when used for the purpose of early detection. On the other hand, although barium enemas are able to detect largely formed advanced cancer, it has the disadvantage of having difficulty in detecting small lesions.

In addition, since endoscopic examinations allow visualization of an affected site directly, the examination results are highly reliable and these examinations have been shown to contribute to reductions in the mortality rate and incidence of colorectal cancer. However, since the affected area may be extremely small in the early stages of cancer, there is the problem of such affected areas being difficult to detect by endoscopic examination.

In this manner, since detection techniques for effectively detecting groups at high risk to colorectal cancer and early colorectal cancer have yet to be fully developed, there are many cases in which diagnosis is made only after the disease stage has advanced to a certain degree. Therefore, there is a desire for a highly sensitive and highly specific testing method that enables risk assessment and discovery of colorectal cancer at an early stage either lowly invasively or non-invasively.

A method that is currently attracting attention as a method for detecting colorectal cancer from the stage of an early lesion consists of analyzing at the molecular level using nucleic acid and protein analysis techniques. For example, a genetic predisposition to familial adenomatous polyposis (FAP) is a typical example of a risk factor of colorectal cancer, and the risk of colorectal cancer can be accessed by conducting a genetic analysis on the subject. More recently, among groups having and not having a characteristic genetic predisposition in the manner of FAP, age (50 years or older) and lifestyle factors such as obesity, alcohol consumption and smoking are known to increase the future risk of colorectal cancer. Consequently, molecular abnormalities (epigenetics, expression abnormalities) attributable to lifestyle are attracting attention as a way of predicting future colorectal cancer. In actuality, numerous molecules have been found during the course of genome-wide association studies (GWAS) that are suggested to be involved in colorectal cancer.

Technologies for analyzing nucleic acids present in stool and blood are being developed for the purpose of determining the presence of molecular abnormalities in the large intestine. However, since nucleic acids originating in extremely small lesions are only present in trace amounts, early stage detection of molecular abnormalities is difficult. The reflection of changes in microlesions measuring 1 mm or less in the blood is also difficult in terms of the sensitivity of analytical devices. In addition, stool contains a large amount of intestinal bacteria as well as epithelial cells that have exfoliated from regions other than lesions, and there is a large amount of noise. Consequently, in order to detect early stage colorectal adenomas or colorectal tumors when using stool for the specimen, a superior molecular marker is required that increases in expression level to a greater degree than in normal tissue at an early stage of canceration and tumor development. Thus, the development of a technology for early risk assessment of colorectal cancer by detecting molecular abnormalities at an early stage in the large intestine has yet to be realized.

On the other hand, aberrant crypt foci (ACF) were first reported by Bird, et al. in 1987 as microlesions that are densely stained with methylene blue in the large intestine of rats administered a carcinogenic substance (azoxymethane). ACF constitute the first morphological abnormality that can be detected morphologically (see, for example, Non-Patent Document 1), and since accelerated cell proliferation activity and K-ras mutations are also observed, ACF have been suggested to be involved in the onset of colorectal cancer and colorectal adenoma. Lesions densely stained with methylene blue have also been observed in excised specimens of human large intestine in cancer patients and patients with polyps, and the amount of these lesions has been reported to increase in the order of healthy persons, patients with polyps and cancer patients (see, for example, Non-Patent Document 2). On the basis of these findings, ACF is being more frequently used as an indicator in colorectal cancer preventive research.

ACF measuring 1 mm or less in size are difficult to be detected in ordinary endoscopic examinations, and are generally detected using a magnifying endoscope. However, since the use of a magnifying endoscope requires considerable time for the procedure, the opportunities for its use are limited and it is difficult to be used for primary screening of early colorectal cancer. In this manner, development of a technology for evaluating the risk of colorectal cancer by detecting micro ACF microscopically or endoscopically has yet to be realized.

In addition, searches have been made for useful molecular markers in order to analyze ACF at the molecular level. More specifically, reported examples of molecules for which expression levels fluctuate in ACF include cyclin D1, cyclooxygenase 2 (COX2), catenin beta 1 (β catenin), nitric oxide synthase 2, inducible (iNOS); epidermal growth factor receptor (EGFR) and CD44 molecule (CD44). However, each of these markers has been reported on the basis of small-scale studies, and differing from colorectal cancer, there have been no reports relating to evaluation of large-scale studies regarding molecular alterations in ACF lesions.

For example, in an example of prior research on COX2 (see Non-Patent Document 3), since molecular alterations were analyzed using samples collected throughout the large intestine, specificity for ACF was low and molecular alterations that can be ascribed only to ACF were not analyzed in comparison to the surrounding (normal) tissue. In addition, in prior research on cyclin D1 (see Non-Patent Document 4), prior research on iNOS (see Non-Patent Document 5) and prior research on CD44 (see Non-Patent Document 6), since only immunostaining data was analyzed, this research lacks quantitativeness and specificity and has not been able to be applied to actual medical treatment. Moreover, in prior research on iNOS (see Non-Patent Document 7) and prior research on EGFR (see Non-Patent Document 8), analyses were targeted at comparatively large ACF of 50 crypts, while analyses were not conducted on molecular alterations of early stage micro ACF. In this manner, although several molecules are known which have been suggested as having the possibility of being able to be used as ACF marker molecules, none of these are considered to be reliable enough to warrant clinical application.

In other words, the analysis of molecules demonstrating alterations in expression level at the stage of microlesions typically represented by micro ACF measuring 1 mm or less (namely, at an early stage), and the simple assessment of the future risk of colorectal cancer by molecular biological determination, have yet to be realized.

### Prior Art Documents

### Patent Documents

Patent Document 1: Japanese Unexamined Patent Application, First Publication No. 2007-229054

### Non-Patent Documents

Non-Patent Document 1: Kelloff, et al., 2006, Clinical Cancer Research, Vol. 12, No. 12, pp. 3661-3697.
Non-Patent Document 2: Takayama, et al., The New England Journal of Medicine, 1998, Vol. 339, No. 18, pp. 1277-1284.
Non-Patent Document 3: Fichera, et al., Journal of Surgical Research, 2007, Vol. 142, pp. 239-245.
Non-Patent Document 4: Paulsen, et al., Cancer Research, 2005, Vol. 65, pp. 121-129.
Non-Patent Document 5: Xu, et al., World Journal of Gastroenterology, 2003, Vol. 9, No. 6, pp. 1246-1250.
Non-Patent Document 6: Boon, et al., Cancer Research, 2002, Vol. 62, pp. 5126-5128.
Non-Patent Document 7: Takahashi, et al., Carcinogenesis, 2000, Vol. 21, No. 7, pp. 1319-1327.
Non-Patent Document 8: Cohen, et al., Cancer Research, 2006, Vol. 66, pp. 5126-5128.

### DISCLOSURE OF THE INVENTION

### Problems to be Solved by the Invention

An object of the present invention is to provide a method for detecting ACF by analyzing a test region of large intestine tissue at the molecular level.

### Means for Solving the Problems

As a result of conducting extensive research to solve the aforementioned problems, the inventors of the present invention found that expression levels of GSTp, iNOS, CD44, EGFR, COX2 and Fzd1 increased more in micro ACF measuring 1 mm or less than in normal tissue, thereby leading to completion of the present invention.

Namely, the present invention provides:
(1) a method for detecting aberrant crypt foci (ACF), comprising: detecting one or more types of molecules for which ACF-specific expression increases selected from the group consisting of GSTp, iNOS, CD44, EGFR, COX2 and Fzd1 present in a test region of large intestine tissue;
(2) the ACF detection method described in (1) above, wherein the diameter of the test region is 0.5 mm or less;
(3) the ACF detection method described in (1) or (2) above, wherein the test region includes a region where ACF are suspected;
(4) the ACF detection method described in (3) above, comprising comparing the amount of molecules for which ACF-specific expression increases in the test region with the amount of molecules for which ACF-specific expression increases in a region of normal tissue in the same large intestine tissue as the test region;
(5) the ACF detection method described in any of (1) to (4) above, wherein the test region is a specimen collected from the body;
(6) the ACF detection method described in any of (1) to (4) above, wherein detection of the molecules for which ACF-specific expression increases is carried out in vivo;
(7) the ACF detection method described in any of (1) to (6) above, wherein the molecules for which ACF-specific expression increases are detected by fluorescent labeling;
(8) the ACF detection method described in any of (1) to (7) above, wherein the molecules for which ACF-specific expression increases are mRNA or proteins;
(9) an ACF detection marker, which is GSTp, iNOS, CD44, EGFR, COX2 or Fzd1; and
(10) a method for evaluating risk of colorectal cancer and colorectal adenoma in subjects based on the results of detecting ACF in a test region of large intestine tissue of the subjects using the ACF detection method described in any of (1) to (8) above.

### Effects of the Invention

According to the ACF detection method of the present invention, ACF can be detected using a molecular biological technique. In particular, the ACF detection method of the present invention is able to accurately detect micro ACF measuring 1 mm or less.
Since ACF are considered to be indicators of colorectal cancer and colorectal adenoma, the ACF detection method of the present invention is useful for early detection of colorectal cancer and colorectal adenoma, and the assessment of the risk thereof.

### BRIEF DESCRIPTION OF THE DRAWINGS

FIG. 1 depicts graphs showing the relative expression levels of GSTp in control samples (samples of normal surrounding tissue) and ACF samples (samples of ACF sites) from rat individuals in Example 1.
FIG. 2 depicts graphs showing the relative expression levels of iNOS in control samples and ACF samples from rat individuals in Example 1.
FIG. 3 depicts graphs showing the relative expression levels of EGFR in control samples and ACF samples from rat individuals in Example 1.
FIG. 4 depicts graphs showing the relative expression levels of CD44 in control samples and ACF samples from rat individuals in Example 1.
FIG. 5 depicts graphs showing the relative expression levels of Fzd1 in control samples and ACF samples from rat individuals in Example 1.
FIG. 6 depicts graphs showing the relative expression levels of Cdh1 in control samples and ACF samples from rat individuals in Example 1.
FIG. 7 depicts graphs showing the relative expression levels of Ctsb in control samples and ACF samples from rat individuals in Example 1.
FIG. 8 depicts graphs showing the relative expression levels of PCNA in control samples and ACF samples from rat individuals in Example 1.
FIG. 9 depicts graphs showing the relative expression levels of Ctnnb in control samples and ACF samples from rat individuals in Example 1.
FIG. 10 depicts graphs showing the relative expression levels of Met in control samples and ACF samples from rat individuals in Example 1.
FIG. 11 depicts graphs showing the relative expression levels of COX2 in control samples and ACF samples from rat individuals in Example 1.
FIG. 12 depicts fluorescent stained images of a GSTp-specific fluorescent probe in an AOM rat in Example 2.
FIG. 13 is a graph showing the results of analyzing fluorescence intensity of an ACF site in a fluorescent stained image of the AOM rat of FIG. 12 and the fluorescence intensity of a normal tissue region of the same area as the ACF site in Example 2.
FIG. 14 depicts fluorescent stained images of a COX2-specific fluorescent probe in an AOM rat and normal rat in Example 2.
FIG. 15 is a graph showing the results of analyzing fluorescence intensity of an ACF site in a fluorescent stained image of the AOM rat of FIG. 14 and the fluorescence intensity of a normal tissue region of the same area as the ACF site in Example 2.
FIG. 16 depicts immunostained images of an AOM rat obtained using fluorescent-labeled anti-GSTp antibody in Example 2.

### EMBODIMENTS FOR CARRYING OUT THE INVENTION

In the present invention and the description of the present application, molecules for which ACF-specific expression increases refer to molecules for which gene expression levels increase in ACF more than in surrounding normal tissue in the large intestine tissue of the same individual.
In addition, in the present invention and the description of the present application, large intestine indicates a region that includes the appendix, colon, rectum and anus, while large intestine tissue indicates tissue that includes large intestine mucous membrane and large intestine epithelium.

In the present invention and description of the present application, the diameter of a region indicates diameter in the case the region is a circle or oval (long axis in the case of an oval), the diameter of an approximate circle when the region is approximated to a circular shape in the case the region is of a shape other than a circle or oval, or the diameter of an approximate oval in the case of having approximated the shape of the region to that of an approximate oval.

The ACF detection method of the present invention is characterized by detecting one of more types of molecules for which ACF-specific expression increases selected from the group consisting of glutathione S-transferase pi (GSTp), iNOS (NOS2), CD44, EGFR, COX2 and frizzled homolog 1 (Fzd1) present in a test region of large intestine tissue. Although GSTp may have a plurality of isoforms, these isoforms are only required to be those that are expressed in large intestine tissue, and only one type of isoform may be detected or a plurality of isoforms may be detected. All of these six types of molecules for which ACF-specific expression increases are molecules for which expression levels increase in micro ACF measuring 1 mm or less more than in surrounding normal tissue. Consequently, these six types of molecules for which ACF-specific expression increases are clinically useful marker molecules for detecting ACF and particularly for detecting micro ACF, and by using the expression levels of these molecules for which ACF-specific expression increases as indicators, comparatively large ACF (namely, ACF in which morphological abnormalities have progressed) as well as early micro ACF can be accurately detected.

In the ACF detection method of the present invention, at least one type of the aforementioned six types of molecules for which ACF-specific expression increases may be detected, or two or more types may be detected for a single test region.

Although there are no particular limitations on the test region where expression levels of molecules for which ACF-specific expression increases are detected provided it is a region of large intestine tissue, it is preferably a region where ACF are suspected (suspect ACF region). An example of a suspect ACF region is a region of large intestine tissue densely stained by methylene blue stain. Although regions other than ACF also end up being stained by methylene blue staining, in the ACF detection method of the present invention, ACF can be accurately detected by methylene blue staining by using the expression levels of molecules for which ACF-specific expression increases as indicators. Other examples of suspect ACF regions include regions in which morphological abnormalities are observed by endoscopic observation, microscopic observation or image analysis and the like.

The six types of molecules for which ACF-specific expression increases targeted for detection in the present invention are all molecules for which gene expression levels increase in micro ACF more than in normal tissue. Consequently, in the present invention, micro ACF can be detected by using a region containing a suspect ACF region having a diameter of 1 mm or less as a test region.

There are no particular limitations on the size of the test region used in the ACF detection method of the present invention, and although the size of the test region can be suitably determined in consideration of such factors as the size of the suspect ACF region, the suspect ACF region preferably occupies a large proportion of the test region. In the case a normal tissue region occupies an excessively large proportion of the test region, even if the suspect ACF region is actually a region in which ACF are present, it becomes difficult to detect a difference between the amount of molecules for which ACF-specific expression increases in the test region and the amount of molecules for which ACF-specific expression increases in normal tissue. For example, in the case the test region contains a suspect ACF region having a diameter of 1 mm or less, the diameter of the test region is preferably 1 mm or less, more preferably less than 1 mm and even more preferably 0.5 mm or less.

The molecules for which ACF-specific expression increases that are detected in the ACF detection method of the present invention are only required to be molecules that reflect gene expression level, and may be mRNA or proteins. Namely, the ACF detection method of the present invention enables detection of ACF in a test region by acquiring information at the RNA level or protein level on molecules for which ACF-specific expression increases in the test region.

The method used to detect each molecule for which ACF-specific expression increases is only required to be a method in which detection results are dependent on the amount or concentration of each molecule in the test region, and can be suitably selected and used from among known methods used to detect mRNA or protein in a specimen. In particular, the methods used to detect molecules for which ACF-specific expression increases are preferably carried out with a method used for expression analysis. Each method can be carried out in accordance with ordinary methods.

In the case ACF are contained in a test region, the amount of molecules for which ACF-specific expression increases in the test region increases more than in normal tissue in large intestine tissue. Consequently, ACF can be detected in a test region by comparing the amount of molecules for which ACF-specific expression increases present in the test region with the number of molecules for which ACF-specific expression increases present in normal tissue in large intestine tissue. Namely, in the case the amount of molecules for which ACF-specific expression increases in a test region is greater than that in normal tissue, ACF can be judged to be contained in the test region, while in the case that amount is equal to or lower than the amount in normal tissue, ACF can be judged to not be contained in the test region. Comparison of the amounts of molecules for which ACF-specific expression increases between a test region and a normal tissue region may be carried out per unit surface area or unit volume of each region, or may be carried out per unit amount of nucleic acid or unit amount of protein contained in each region.

In the present invention, a test region and normal tissue surrounding that test region are preferably compared in large intestine tissue of the same individual. Although there are individual differences in the expression level of each molecule for which ACF-specific expression increases, effects attributable to individual differences can be eliminated by comparing within the same individual.

Molecules for which ACF-specific expression increases may not be detected in normal tissue and may only be detected in ACF depending on the type and sensitivity of the detection method used. In this case, ACF are judged to be contained in a test region in the case molecules for which ACF-specific expression increases were detected in the test region, while ACF are judged to not be contained in the test region in the case molecules for which ACF-specific expression increases were not detected.

In the case a molecule for which ACF-specific expression increases is RNA, examples of methods used to detect each molecule for which ACF-specific expression increases include a method consisting of utilizing a nucleic acid amplification reaction using primers specific to each molecule, and a method consisting of utilizing a hybridization reaction using probes specific for each molecule. An example of a method that utilizes a nucleic acid amplification reaction consists of detecting a molecule for which ACF-specific expression increases in a test region, or quantitatively measuring the amount thereof in a normal tissue region to a degree that allows comparison, by synthesizing cDNA from RNA contained in the test region by a reverse transcription reaction followed by carrying out a nucleic acid amplification reaction such as RT-PCR using the resulting cDNA as a template. Extraction of RNA from the test region, the reverse transcription reaction, and the RT-PCR or other nucleic acid amplification reaction can be suitably selected and carried out from among techniques known in the applicable technical fields.

When using a method that utilizes a nucleic acid amplification reaction, an amplified molecule for which ACF-specific expression increases can be fluorescently labeled and quantitatively detected by using a fluorescent intercalator or primer labeled with a fluorescent substance. On the other hand, when using a method that utilizes hybridization, a molecule for which ACF-specific expression increases can be fluorescently labeled and quantitatively detected in a test region by using a probe labeled with a fluorescent substance or a probe that has been modified so as to emit fluorescent light only after having hybridized.

In the case a molecule for which ACF-specific expression increases is a protein, each molecule for which ACF-specific expression increases can be detected by an immunochemical technique using an antibody that specifically recognizes each molecule (specific antibody). More specifically, after bonding a specific antibody of each molecule labeled with a marker to the molecule present in a test region, a molecule for which ACF-specific expression increases can be fluorescently labeled and quantitatively detected in the test region by measuring a signal from the marker. Labeling with a marker may be carried out by bonding the marker directly to a specific antibody of each molecule, or by bonding to a secondary antibody that specifically bonds with that specific antibody. The marker can be suitably selected and used from among markers commonly used in antigen-antibody reactions or those used when detecting for the presence or absence of bonding of two molecules. Examples of such markers include fluorescent substances, magnetic substances and radioisotopes. Fluorescent substances are preferably used as markers from the viewpoints of high sensitivity and safety. The antigen-antibody reaction can be carried out in accordance with ordinary methods. In addition to immunochemical techniques, a probe that specifically indicates activity of a molecule for which ACF-specific expression increases can be used, and a molecule for which ACF-specific expression increases in the form of a protein can be detected by detecting the probe.

Detection of a molecule for which ACF-specific expression increases can be carried out on a specimen collected from the body. For example, a sample of a test region containing a suspect ACF region can be collected microscopically from a sample obtained by excising large intestine tissue collected by surgically excising a partial region of large intestine tissue of the body. At this time, a sample of a normal tissue region, and preferably normal tissue surrounding the test region, is collected from the same surgically excised large intestine tissue sample as necessary. In addition, a sample of a test region (biopsy sample) can be collected directly from the body by preliminarily staining large intestine tissue of the body with methylene blue and then surgically excising the densely stained region. A sample of normal tissue surrounding the test region can also be collected from the body in the same manner as the surgically excised sample of large intestine tissue. Samples of a test region and normal tissue region collected in this manner are then used to detect molecules for which ACF-specific expression increases. Methylene blue staining of large intestine tissue in vivo can be carried out in accordance with ordinary methods.

Detection of a molecule for which ACF-specific expression increases can also be carried out in vivo. For example, a labeled probe specific for a molecule for which ACF-specific expression increases, a specific antibody of a molecule for which ACF-specific expression increases labeled directly or indirectly, or a specific labeled probe that indicates activity of a molecule for which ACF-specific expression increases, is coated or sprayed onto a region containing a test region in the large intestine of the body, and after allowing the probe or specific antibody to bond to the molecule for which ACF-specific expression increases to label the molecule for which ACF-specific expression increases, the molecule for which ACF-specific expression increases can be detected by detecting the label.

In the case of detecting a molecule for which ACF-specific expression increases in vivo, the molecule for which ACF-specific expression increases is preferably detected by fluorescent labeling. More specifically, a molecule for which ACF-specific expression increases is first fluorescently labeled using a probe or specific antibody labeled with a fluorescent substance. Subsequently, fluorescence emitted from the label is optically detected using an endoscope or gastrointestinal video scope to obtain a fluorescent image. A molecule for which ACF-specific expression increases can then be detected both with high sensitivity and quantitatively by analyzing the resulting fluorescent image.

Detection of a molecule for which ACF-specific expression increases in vivo can be carried out easily and efficiently by using a fluorescent endoscope. More specifically, an endoscopy system can be used, for example, that is partially inserted into a body cavity of the body to acquire images of an imaging target within the body cavity, and is provided with agent discharge means for discharging toward the imaging target a sensitive fluorescent agent that bonds or reacts with a specific substance present in the imaging target or a fluorescent agent that is accumulated in the imaging target, discharge control means for controlling the agent discharge means, a light source unit that emits excitation light for exciting the fluorescent agent and irradiation light having spectral properties that differ from those of the excitation light, optics for allowing the excitation light and irradiation light from the light source unit to propagate towards the imaging target, and imaging means provided at a site that is inserted into the body cavity and is capable of capturing images of fluorescent light radiated from the imaging target due to the excitation light and light of a frequency band differing from the fluorescent light radiated from the imaging target due to the irradiation light (see, Japanese Unexamined Patent Application, First Publication No. 2007-229054). A probe or specific antibody specific for a molecule for which ACF-specific expression increases that has been labeled with a fluorescent substance is used for the fluorescent agent.

In the ACF detection method of the present invention, the sample provided for detection of a molecule for which ACF-specific expression increases is only required to be that which originates in the large intestine of an animal, and may be a sample from a fish, bird, reptile or mammal. In the present invention, the sample is preferably from a mammal. Examples of mammals include rodents such as mice, rats or guinea pigs, animals other than humans such as dogs, cats, cows, horses, sheep, pigs or monkeys, and humans.

Information as to whether or not the amount of a molecule for which ACF-specific expression increases in a test region is greater than the amount in normal tissue is useful when judging whether or not ACF are present in the test region. Accordingly, detection results obtained according to the ACF detection method of the present invention are useful as information provided for diagnosing ACF.

In addition, since the expression levels of the six types of molecules for which ACF-specific expression increases in the present invention indicate the presence of ACF, and since ACF have a high probability of progressing to colorectal cancer in the future, detection results obtained according to the ACF detection method of the present invention serve as extremely useful information when assessing the risk of existing colorectal cancer, and during lowly invasive assessment of the risk of future colorectal cancer at an early stage. For example, according to the ACF detection method of the present invention, in the case in which the amount of a molecule for which ACF-specific expression increases in a test region is greater than the amount in a surrounding normal tissue region in large intestine tissue of a subject, and ACF have been detected in the test region, the subject can be assessed has having a high risk of the onset of colorectal cancer and colorectal adenoma in the future. Conversely, in the case the amount of a molecule for which ACF-specific expression increases in a test region is equal to or less than the amount in a surrounding normal tissue region, and ACF is not detected in the test region, the subject can be assessed has having a low risk for the onset of colorectal cancer and colorectal adenoma in the future.

### Examples

Although the following provides a more detailed explanation of the present invention by indicating examples thereof, the present invention is not limited to the following examples.

### [Example 1]

The gene expression levels of all 11 types of candidate molecules consisting of GSTp1, iNOS (NOS2), CD44, EGFR, Fzd1, Cdh1 (cadherin1), Ctsb (cathepsin B), PCNA (proliferating cell nuclear antigen), Ctnnb1 (catenin beta), Met (met proto-oncogene) and COX2 were compared between a surrounding normal tissue region and a suspect ACF region in the same individual to identify those candidate molecules for which gene expression levels increased specifically for ACF.
More specifically, a sample collected only from a region confirmed microscopically to an ACF site and a sample collected only from a surrounding region of normal tissue were respectively prepared from excised large intestine tissue samples collected from rats in which the formation of ACF was induced in large intestine tissue by administration of azoxymethane (AOM), and the expression levels of each molecule were measured and compared. The following provides a description of the details thereof.

Five F344/N rats (males, age 4 weeks) were subcutaneously administered AOM (15 mg/kg) once a week for three weeks following a one week acclimation period. The large intestines were excised from each of the rats over the course of 11 to 14 weeks after the final administration to obtain ACF samples. More specifically, the excised rat large intestines were washed with cold PBS to remove the contents thereof, and after opening in the longitudinal direction, the tissues were fixed using a solution of 60% methanol and 10% acetic acid. Following fixation, the surface layer of the intestinal mucosa was stained with 0.2% methylene blue. Those regions containing sites that were microscopically confirmed to be ACF sites were collected from the stained rat large intestines for use as ACF samples. At this time, micro ACF measuring 1 mm or less were selected and collected. Surrounding normal tissue was simultaneously collected for use as control samples. ACF samples and control samples were collected from two of the rats (Rats 1 and 2) using a commercially available biopsy instrument of the smallest size, and from the remaining three rats (Rats A to C) using a homemade biopsy instrument having a diameter of less than 0.5 mm.

Total RNA was extracted from the resulting ACF samples and control samples using the PAXgene Tissue RNA Kit (QIAGEN Inc.), and the quality of the extracted RNA was confirmed using a bioanalyzer (Agilent Technologies Inc.). An RT reaction was carried out for 60 minutes at 37°C in a reaction solution (final volume: 20 µL) to which had been added RNA of RIN7 or more (5 ng) to synthesize cDNA. For the pre-amplification reaction, a pre-amplification reaction was carried out for a low number of cycles using a primer set for amplifying each candidate molecule by using the resulting cDNA as template. The commercially available primers shown in Table 1 (Applied Biosystems Inc.) were respectively used for the primer sets. More specifically, 10 µL of reaction solution following the RT reaction, 12.5 µL of solution obtained by preliminarily mixing each primer set, 25 µL of nucleic acid amplification reagent (Taqman Gene Expression Master Mix, Applied Biosystems Inc.) and 2.5 µL of ultrapure water were each added to prepare a reaction solution having a final volume of 50 µL. Each reaction solution was placed in a PCR device (Eppendorf Corp.), and after heat treating for 10 minutes at 95°C, PCR was carried out for 14 cycles of a thermal reaction consisting of 15 seconds at 95°C and 4 minutes at 60°C. Following reaction, the reaction liquid was diluted 20 fold and provided for use as real-time PCR sample.

**[Table 1]**

| Product Name | Candidate Molecule | NCBI Location Chromosome |
|---|---|---|
| RN00584431_g1 | Ctnnb1 | (Chr.8-125978161-125987670) |
| Rn00561646_m1 | Nos2 | (Chr.10-65036889-65072771) |
| Rn00681157_m1 | Cd44 | (Chr.3-88022960-88110352) |
| Rn00580398_m1 | Egfr | (Chr.14-97617358-97788213) |
| Rn00580462_m1 | Met | (Chr.4-43134183-43211357) |
| Rn02770492_gH | Gstp1 | (Chr.1-206627464-206629586) |
| Rn00575030_m1 | Ctsb | (Chr.15-42402829-42423701) |
| Rn00673588_mH | Pcna | (Chr.3-120008711-120012673) |
| Rn00580109_m1 | Cdh1 | (Chr.19-36442693-36512091) |
| Rn01755425_s1 | Fzd1 | (Chr.4-25994423-25999379) |
| Rn01483828_m1 | cox2 | (Chr.13-64427166-64435401) |

Real-time PCR was then carried out using the pre-amplified cDNA as template followed by detection of the expression products (mRNA) of each candidate molecule. More specifically, after dispensing 5 µL aliquots of each pre-amplified cDNA into a 0.2 mL 96-well plate, 4 µL of ultrapure water, 10 µL of nucleic acid amplification reagent (Taqman Gene Expression Master Mix, Applied Biosystems Inc.) and 1 µL of primer probe set were added to each well to prepare PCR reaction solutions. The 96-well plate was placed in a real-time PCR device (Applied Biosystems Inc.), and after heat treating for 2 minutes at 50°C and 10 minutes at 95°C, 40 cycles of a thermal reaction consisting of 15 seconds at 95°C and 1 minute at 60°C were carried out, fluorescence intensity was measured over time.

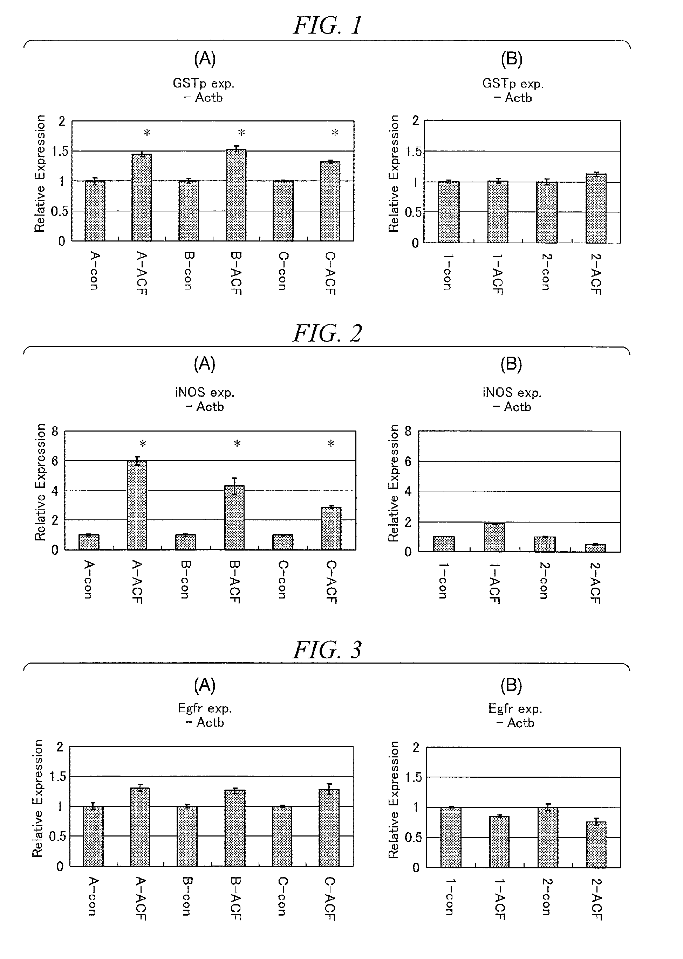

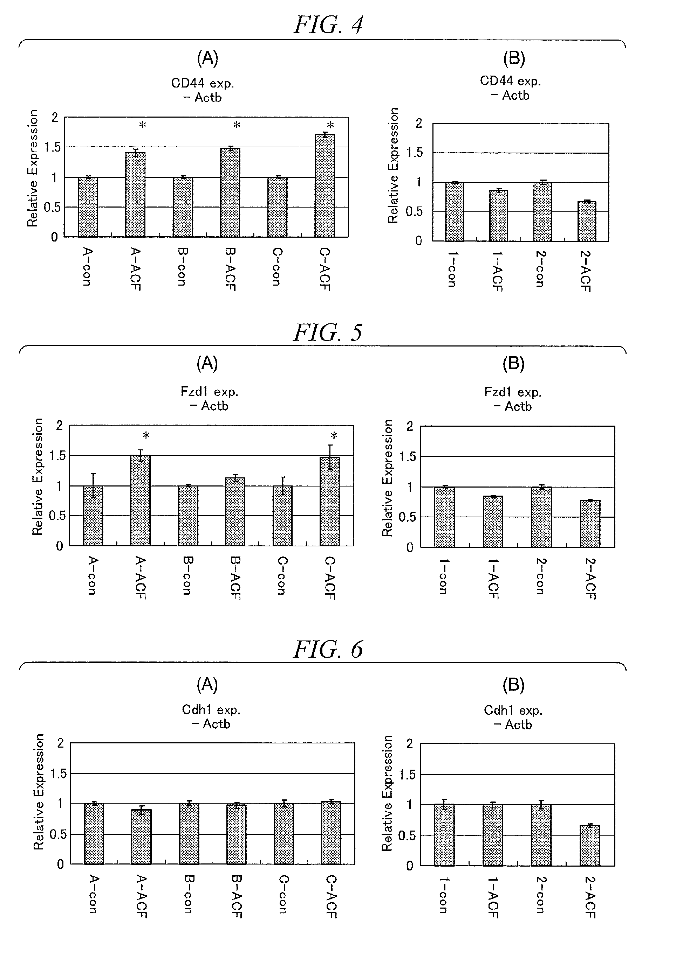

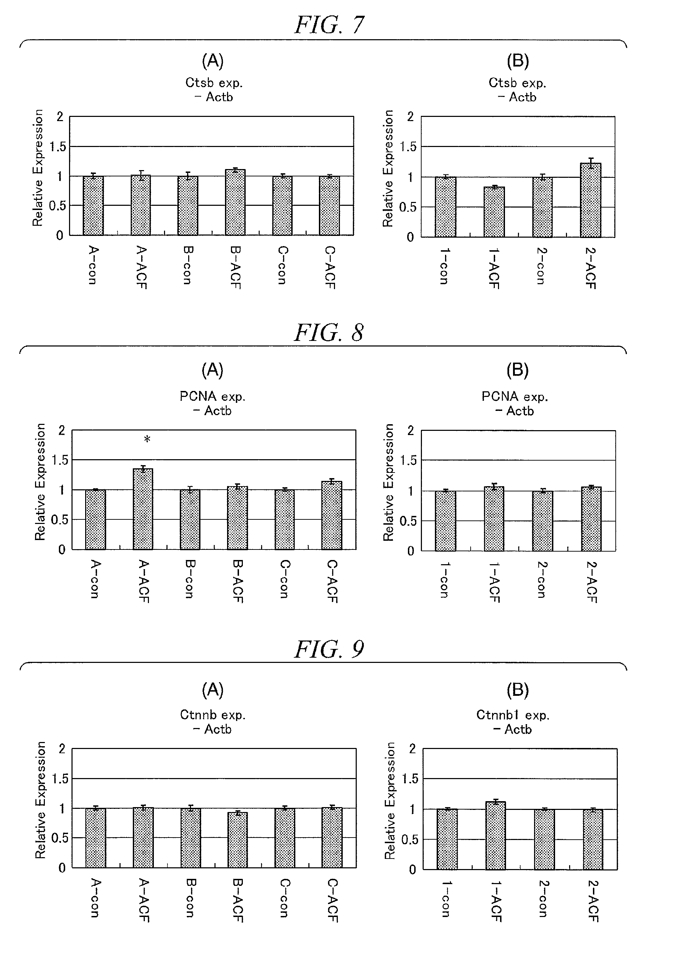

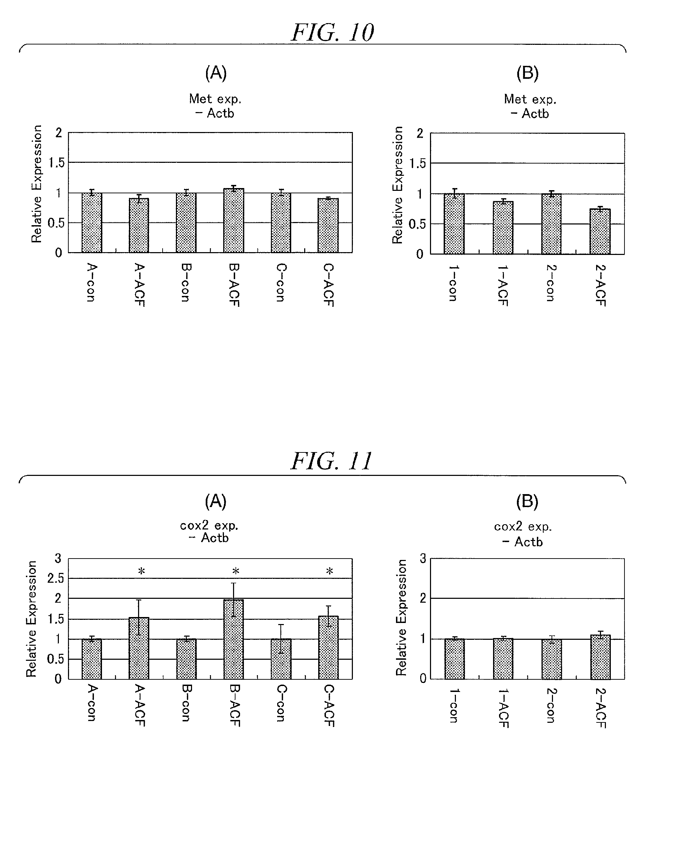

The results of measuring fluorescence intensity were analyzed, and the gene expression levels of the candidate molecules in the RNA recovered from each sample were calculated. The relative values of gene expression levels in the ACF samples collected from the same individual were calculated based on a value of 1 for gene expression levels in the control samples. The results of the calculations for each candidate molecule are respectively shown in FIGS. 1 to 11. In each of the graphs, (A) indicates the results of sampling using the homemade biopsy instrument having a diameter of less than 0.5 mm, while (B) indicates the results of sampling using the commercially available biopsy instrument having a diameter of 1 mm. In addition, in each of the graphs, "X-con" indicates the results for the control sample of Rat X, while "X-ACF" indicates the results for the ACF sample of Rat X. In addition, those samples for which the relative expression level of the ACF sample was 1.3 or more are indicated with asterisks (*).

As a result, when comparisons were made between expression levels in surrounding normal tissue and expression levels in ACF in the same individual, in the case of using the homemade biopsy instrument having a diameter of less than 0.5 mm, expression levels were increased in the ACF lesions (ACF samples) in comparison with surrounding normal tissue (control samples) in all three rat individuals for the six types of molecules of GSTp1, iNOS, CD44, EGFR, COX2 and Fzd1. In other words, these six types of molecules were determined to demonstrate increased gene expression levels specific for ACF.

On the other hand, in the case of sampling using the commercially available biopsy instrument having a diameter of 1 mm, there were no differences in gene expression levels observed between ACF lesions and surrounding normal tissue. On the basis of these results, in the case of detecting micro ACF of 1 mm or less, analysis of ACF-specific gene expression was suggested to only be possible in the case of collecting samples using a biopsy instrument having a diameter of less than 0.5 mm, which is smaller than that used in conventional methods.

### [Example 2]

Fluorescent probes that specifically react with each molecule were respectively prepared for the six types of molecules of GSTp1, iNOS, CD44, EGFR, COX2 and Fzd1 for which expression levels specific for ACF lesions were observed to increase in Example 1, and reactivity with surrounding normal tissue and that in suspect ACF regions were compared in the same individual for each of the fluorescent probes.
More specifically, the aforementioned probes were sprayed onto excised large intestine tissue excised from rats in which formation of ACF was induced in large intestine tissue by administration of azoxymethane (AOM), and together with confirming ACF sites microscopically, fluorescence was observed with a fluorescence microscope. The following provides a description of the details thereof. Furthermore, a fluorescent probe that reacts with all isoforms of GSTp was used for GSTp1.

Five F344/N rats (males, age 4 weeks) were subcutaneously administered AOM (15 mg/kg) once a week for three weeks following a one week acclimation period. The large intestines were excised from each of the rats over the course of 7 to 10 weeks after the final administration followed by evaluation with the fluorescent probes. More specifically, the excised rat large intestines were washed with cold PBS to remove the contents thereof, and after opening in the longitudinal direction, the intestines were sprayed with a fluorescent probe that specifically reacts with each molecule. The stained rat large intestines were observed with a fluorescence microscope, and together with confirming a reaction in a suspect ACF region (fluorescence intensity), ACF lesions in the same region were confirmed with a stereoscopic microscope. Similar observations were simultaneously carried out for a control group subcutaneously administered PBS. The probes described in Reference A (JACS 2008, Vol. 130, pp. 14533-14543) and Reference B (WO 2007/149456) as well as probes fluorescently labeled with commercially available antibodies were used for the fluorescent probes.

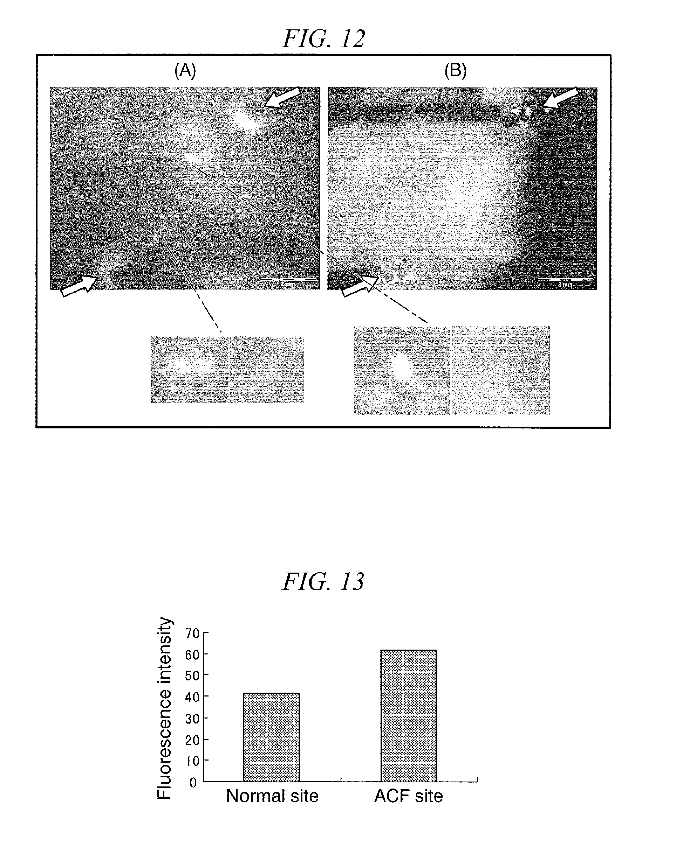

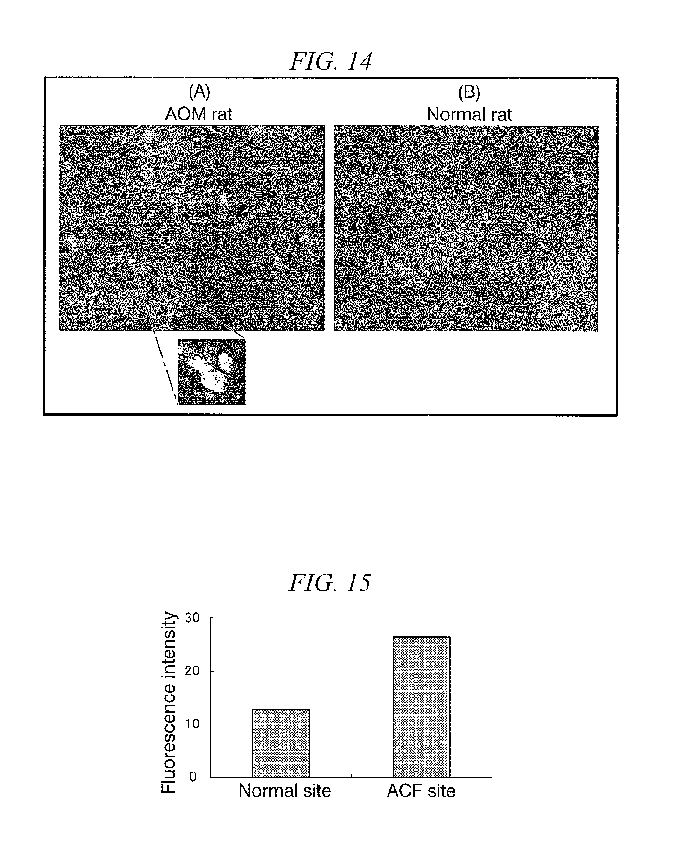

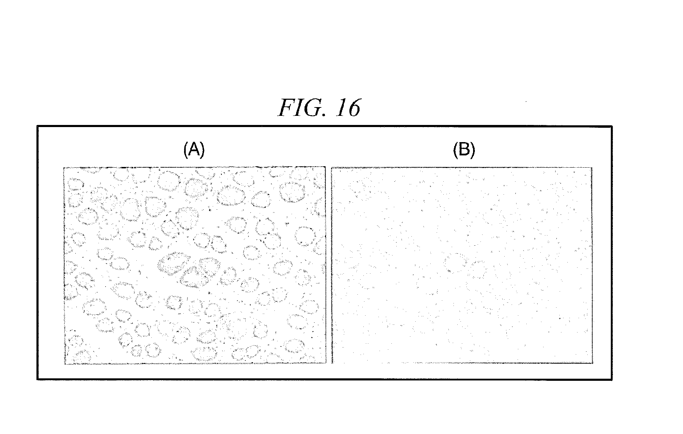

The fluorescent stained images and results of analyzing fluorescence intensity are shown in FIGS. 12 to 15. FIG. 12 (A) shows a fluorescent stained image of a GSTp-specific fluorescent probe described in Reference A, while FIG. 12(B) shows a white light observation image of the same field as FIG. 12 (A). The light transmission image of FIG. 12 (B) was obtained by blacking out sites other than the tissue section by image processing. In addition, in FIGS. 12(A) and 12(B), those locations indicated by arrows indicate the locations of pins used to immobilize the tissue sections. In the fluorescent stained image of an AOM rat of FIG. 12 (A), enlarged images are shown with the transmission light image for two ACF sites (two regions densely stained by the fluorescent probe). FIG. 13 shows the results of analyzing fluorescence intensity of an ACF site in the fluorescent stained image of the AOM rat of FIG. 12 and fluorescence intensity of a normal tissue region of the same area as the ACF site by image processing. FIG. 14 depicts fluorescently stained images of a COX2-specific fluorescent probe described in Reference B. FIG. 14(A) is a fluorescent stained image of an AOM rat, while FIG. 14 (B) is a fluorescent stained image of a normal rat. In the fluorescent stained image of the AOM rat of FIG. 14 (A), an enlarged image of an ACF site (region densely stained by fluorescent probe) is also shown. FIG. 15 shows the results of analyzing fluorescence intensity of the ACF site in the fluorescent stained image of the AOM rat of FIG. 14 (A) and the fluorescence intensity of a normal tissue region of the same area as the ACF site by image processing. Moreover, FIG. 16 depicts immunostained images of an AOM rat obtained using commercially available anti-GSTp antibody. FIG. 16(A) is an HE stained image, while FIG. 16(B) is an immunostained image using anti-GSTp antibody for a continuous section of the same tissue block as that of FIG. 16(A). The comparatively densely stained region located in the center of the HE stained image of FIG. 16(A) is an ACF site.

As a result, fluorescence intensity at ACF sites within the same individual were observed to have relative values that were clearly higher by 1.4 times or more than fluorescence intensity in surrounding normal tissue (FIGS. 13 and 15). In addition, signal sites like those observed in the AOM rats were not observed in the normal rats (control group) (FIG. 14). Moreover, when protein expression at ACF sites confirmed by HE staining in the AOM rats was confirmed by immunostaining, they were confirmed to be more densely stained in comparison with surrounding normal sites (FIG. 16). On the basis of these results, GSTp was clearly determined to be more highly expressed at ACF sites than at surrounding normal sites.

### INDUSTRIAL APPLICABILITY

According to the ACF detection method of the present invention, since ACF can be detected more accurately using a molecular biological technique, this method can be used not only in academic research, but also in fields such as clinical testing for diagnosis of colorectal cancer and colorectal adenoma.

## Claims

1. A method for detecting aberrant crypt foci (ACF), comprising:
detecting one or more types of molecules for which ACF-specific expression increases selected from the group consisting of GSTp, iNOS, CD44, EGFR, COX2 and Fzd1 present in a test region of large intestine tissue.

2. The ACF detection method according to claim 1, wherein the diameter of the test region is 0.5 mm or less.

3. The ACF detection method according to claim 1 or 2, wherein the test region includes a region where ACF are suspected.

4. The ACF detection method according to claim 3, comprising comparing the amount of molecules for which ACF-specific expression increases in the test region with the amount of molecules for which ACF-specific expression increases in a region of normal tissue in the same large intestine tissue as the test region.

5. The ACF detection method according to any of claims 1 to 4, wherein the test region is a specimen collected from the body.

6. The ACF detection method according to any of claims 1 to 4, wherein detection of the molecules for which ACF-specific expression increases is carried out in vivo.

7. The ACF detection method according to any of claims 1 to 6, wherein the molecules for which ACF-specific expression increases are detected by fluorescent labeling.

8. The ACF detection method according to any of claims 1 to 7, wherein the molecules for which ACF-specific expression increases are mRNA or proteins.

9. An ACF detection marker, which is GSTp, iNOS, CD44, EGFR, COX2 or Fzd1.

10. A method for evaluating risk of colorectal cancer and colorectal adenoma in subjects based on the results of detecting ACF in a test region of large intestine tissue of the subjects using the ACF detection method according to any of claims 1 to 8.
